# EUROPEAN PATENT SPECIFICATION

(11) **EP 2 526 854 B1**
(45) Date of publication and mention of the grant of the patent: **18.05.2016**
(21) Application number: 12169077.0
(22) Date of filing: 23.05.2012
(51) Int. Cl.: A61B 1/04, A61B 1/06, A61B 1/00, A61B 5/00

(54) **Endoscope system and method for assisting in diagnostic endoscopy**
Endoskopsystem und Verfahren zur Unterstützung bei der diagnostischen Endoskopie
Système d'endoscope et procédé d'aide au diagnostic en endoscopie

(30) Priority: 24.05.2011 JP 2011115884
(43) Date of publication of application: 28.11.2012
(73) Proprietor: Fujifilm Corporation, Minato-ku Tokyo (JP)
(72) Inventor: Ozawa, Satoshi, Ashigarakami-gun Kanagawa (JP); Saito, Maki, Ashigarakami-gun Kanagawa (JP)
(74) Representative: Klunker . Schmitt-Nilson . Hirsch

(56) References cited:
- US-A1- 2008 177 140
- US-A1- 2011 118 547
- US-B1- 6 826 424

## Description

### BACKGROUND OF THE INVENTION

### 1. Field of the invention

The present invention relates to an endoscope system for automatically detecting a lesion, for example, cancer, and a method for assisting in diagnostic endoscopy.

### 2. Description Related to the Prior Art

Cancer diagnoses using endoscopes have been common in the medical field. In a cancer diagnosis using an endoscope, first, screening for detecting a cancer-suspected lesion is performed when a region of interest is in a far view. Then, the endoscope approaches the cancer-suspected lesion such that the lesion is in a near view. In this state, detailed diagnosis is performed to determine whether the suspected lesion is actually cancer. In the detailed diagnosis, hypertrophy cancer that is cancer increased in size can be easily distinguished under normal observation using white light. However, it is difficult to distinguish the cancer when it resembles inflammation or is embedded in surrounding tissue.

In the screening of the region of interest in the far view, intensity of intrinsic fluorescence emitted from living tissue is observed to pick up a cancer-suspected lesion (see Japanese Patent Laid-Open Publication No. 8-252218). In detailed diagnosis after the screening, narrowband light is applied to the picked-up suspected lesion to enhance the structure of blood vessels relevant to the cancer, for example, that of superficial blood vessels in the image. This facilitates distinguishing the cancer (see Japanese Patent Laid-Open Publication No. 2001-170009).

However, because the intrinsic fluorescence is weak, the detection of the suspected lesion according to the Japanese Patent Laid-Open Publication No. 8-252218 often lacks accuracy, so that a false positive area is detected frequently. This increases redundant detailed diagnoses of the region of interest in the near view, which degrades efficiency in diagnosis.

An endoscope of interest is also disclosed in US 6,826,424 B1.

### SUMMARY OF THE INVENTION

An object of the present invention is to provide an endoscope system and a method for assisting in diagnostic endoscopy for measuring an oxygen saturation level of a cancer-suspected lesion at the time of screening so as to avoid detection of a false positive area and perform cancer diagnosis efficiently.

In order to achieve the above and other objects, the endoscope system of the present invention includes a lighting section, an imaging section, an image processor, a suspected-lesion detection section, an oxygen saturation calculation section, a judging section, and a display section. The lighting section applies illumination light, selected from two or more types of illumination light, to a region of interest. The imaging section images the region of interest illuminated with the illumination light selected. The image processor produces an observation image based on an image signal acquired from the imaging section. The observation image corresponds to the illumination light selected. The suspected-lesion detection section detects a suspected lesion in the observation image. The oxygen saturation calculation section obtains an oxygen saturation level of a blood vessel in the observation image. The judging section judges whether the oxygen saturation level in the suspected lesion is in a hypoxic condition within a predetermined range. The display section displays the observation image.

The lighting section selectively generates one of white light, mixed light in which the white light is mixed with blue narrowband light at a predetermined ratio, and two or more types of narrowband light each having an absorption coefficient varying with the oxygen saturation level, as the illumination light.

The image processor includes a special image processing section for combining a normal light image with a blue-enhanced image to produce a special image in which the blood vessel is enhanced. The normal light image is produced based on the image signal outputted from the imaging section during illumination with the white light. The blue-enhanced image is produced based on the image signal outputted from the imaging section during illumination with the mixed light.

It is preferable that the lighting section alternately generates the white light and the mixed light in respective frames. The imaging section alternately outputs the image signal of the normal light image of one frame and the image signal of the blue-enhanced image of one frame.

It is preferable that the special image processing section performs frequency filtering of a bandwidth ranging from a low frequency to a high frequency to the blue-enhanced image before combining the normal light image with the blue-enhanced image.

It is preferable that the oxygen saturation calculating section acquires two or more oxygen saturation signals from the image signals outputted from the imaging section during the sequential illumination with the respective narrowband light. The image signals correspond to the respective narrowband light. The oxygen saturation level is obtained from magnitude of a ratio between the oxygen saturation signals.

It is preferable that the image processor further includes an oxygen saturation image processing section for producing an oxygen saturation image that is an image of the blood vessel represented by the oxygen saturation level.

It is preferable that the blood vessel in the oxygen saturation image is colored in accordance with the oxygen saturation level.

It is preferable that the endoscope system further includes a display controller for allowing the display section to display the special image before the suspected lesion is detected, and to display the oxygen saturation image when the suspected lesion is judged to be in the hypoxic condition.

It is preferable that the display controller allows the display section to temporarily display the oxygen saturation image between after the suspected lesion is detected and before the suspected lesion is judged to be in the hypoxic condition.

Before the oxygen saturation image is displayed on the display section, it is preferable that the display controller displays a message on the display section. The message notifies that the oxygen saturation image will be displayed on the display section.

The method for assisting in diagnostic endoscopy includes an white light applying step, a normal light image producing step, a mixed light applying step, a blue-enhanced image producing step, a combining step, a detecting step, a narrowband light applying step, a signal acquiring step, an oxygen saturation level obtaining step, an oxygen saturation image producing step, a judging step, and a displaying step. In the white light applying step, white light is applied to a region of interest in a body cavity. In the normal light image producing step, a normal light image is produced based on an image signal outputted from the imaging section during illumination with the white light. In the mixed light applying step, mixed light of the white light and blue narrowband light is applied to the region of interest. The white light and the blue narrowband light is mixed at a predetermined ratio. In the blue-enhanced image producing step, the blue-enhanced image is produced based on an image signal outputted from the imaging section during illumination with the mixed light. In the combining step, the normal light image is combined with the blue-enhanced image to produce a special image in which a blood vessel is enhanced. In the detecting step, a suspected lesion is detected in the special image. In the narrowband light applying step, two or more types of narrowband light is applied sequentially to the region of interest. An absorption coefficient of each narrowband light varies with an oxygen saturation level. In the signal acquiring step, two or more oxygen saturation signals are acquired from image signals, corresponding to the respective narrowband light, outputted from the imaging section during the sequential illumination with the narrowband light. In the oxygen saturation level obtaining step, an oxygen saturation level of blood in the blood vessel is obtained from magnitude of a ratio between the oxygen saturation signals. In the oxygen saturation image producing step, an oxygen saturation image that is an image of the blood vessel represented by the oxygen saturation level is produced. In the judging step, it is judged whether the oxygen saturation level of the suspected lesion is in the hypoxic condition within a predetermined range. In the displaying step, the special image is displayed on a display section before the suspected lesion is detected, and the oxygen saturation image is displayed on the display section when the suspected lesion is judged to be in the hypoxic condition.

It is preferable that the oxygen saturation image is temporarily displayed on the display section between after the suspected lesion is detected and before the suspected lesion is judged to be in the hypoxic condition.

According to the present invention, in the screening, a suspected lesion such as cancer is picked up based on whether the suspected lesion is in the hypoxic condition. This avoids the detection of the false positive area and thus the cancer diagnosis is performed efficiently.

The special image used for detecting the suspected lesion is produced by combining the overall bright normal light image with the blue-enhanced image in which the blood vessels and their structure are enhanced. Accordingly, the suspected lesion is surely detected even if the light quantity is deficient, for example, when the region of interest is in a far view.

### BRIEF DESCRIPTION OF THE DRAWINGS

The above and other objects and advantages of the present invention will be more apparent from the following detailed description of the preferred embodiments when read in connection with the accompanied drawings, wherein like reference numerals designate like or corresponding parts throughout the several views, and wherein:
Fig. 1 is an external view of an endoscope system;
Fig. 2 is a block diagram of the endoscope system;
Fig. 3 is a graph showing emission spectra of first to fourth narrowband light N1 to N4 and phosphor;
Fig. 4 is a front view of a distal portion;
Fig. 5 is a graph showing spectral transmittance of B pixel, G pixel, and R pixel of a color CCD;
Fig. 6 is an explanatory view of an operation of an image sensor in a normal light image signal acquisition frame;
Fig. 7 is a block diagram of a special image processing section;
Fig. 8A is a graph showing a bandwidth of frequency filtering used when a region of interest is in a far view;
Fig. 8B is a bandwidth of frequency filtering used when the region of interest is in a near view;
Fig. 9 is an explanatory view of an operation of the image sensor in the normal light image signal acquisition frame and a first blue-enhanced signal acquisition frame;
Fig. 10 is a graph showing a correlation among signal ratios S2/S1, S3/S1, vascular depth, and oxygen saturation level.
Fig. 11 is an explanatory view of a method for calculating an oxygen saturation level using the correlation shown in Fig. 10;
Fig. 12 is a graph of absorption coefficients of oxyhemoglobin (HbO₂) and deoxyhemoglobin (Hb);
Fig. 13 is an explanatory view of an operation of the image sensor in first to fourth oxygen saturation signal acquisition frames;
Fig. 14 is an explanatory view of an observation distance;
Fig. 15 is an explanatory view showing switching of a display image in a "1-1" special observation mode;
Fig. 16 is an explanatory view showing switching of the display image in a "1-2" special observation mode;
Fig. 17 is an explanatory view showing switching of the display image in a "1-3" special observation mode;
Fig. 18 is an explanatory view showing switching of the display image in a "1-4" special observation mode;
Fig. 19 is an explanatory view showing switching of the display image in a "1-5" special observation mode;
Fig. 20 is an explanatory view showing switching of the display image in a second special observation mode;
Fig. 21 is a graph showing a correlation among the signal ratios B1/G2, R2/G2, and the oxygen saturation level; and
Fig. 22 is an explanatory view of a method for calculating the oxygen saturation level using the correlation shown in Fig. 21.

### DESCRIPTION OF THE PREFERRED EMBODIMENTS

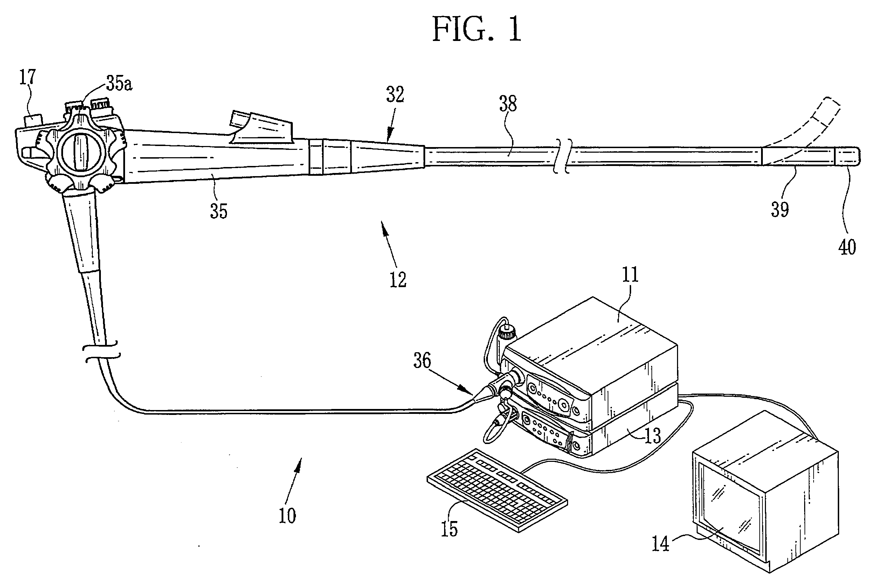

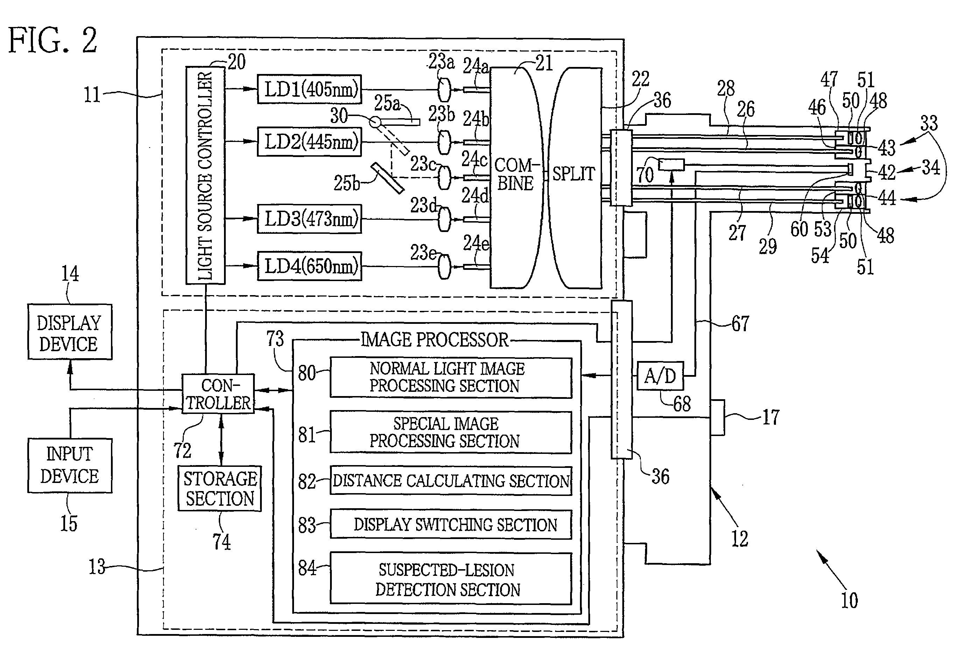

As shown in Figs. 1 and 2, an endoscope system 10 is provided with a light source device 11, an endoscope device 12, a processor device 13, a display device 14, and an input device 15. The light source device 11 generates illumination light. The endoscope device 12 guides the illumination light from the light source device 11 and applies the illumination light to a region of interest in a body cavity (subject). The endoscope device 12 images the region of interest illuminated with the illumination light and acquires an image signal. The processor device 13 performs image processing to the image signal. The display device 14 displays an endoscopic image produced by the image processing. The input device 15 includes a keyboard, for example.

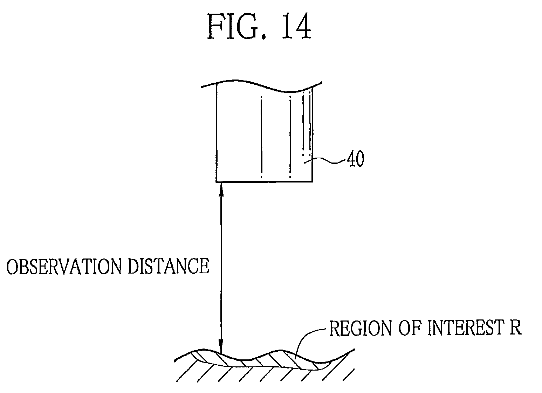

The endoscope system 10 is provided with three modes: a normal observation mode, a first special observation mode, and a second special observation mode. In the normal observation mode, the display device 14 displays a normal light image represented by visible light in a wavelength range from blue to red. In the first special observation mode, a content of a display image is switched between a far view and a near view. The far view refers to a long observation distance (see Fig. 14) between a region of interest "R" of the subject and a distal portion 40 of the endoscope device 12. The near view refers to a short observation distance between the region of interest "R" and the distal portion 40. In the second special observation mode, a suspected lesion, for example, a spot or a brownish area is detected. The first special observation mode is further classified into "1-1" to "1-5" special observation modes depending on a content of image (s) displayed on the display device 14 when the region of interest is in the near view. A selection switch 17 provided on the endoscope device 12 is used for switching between the observation modes.

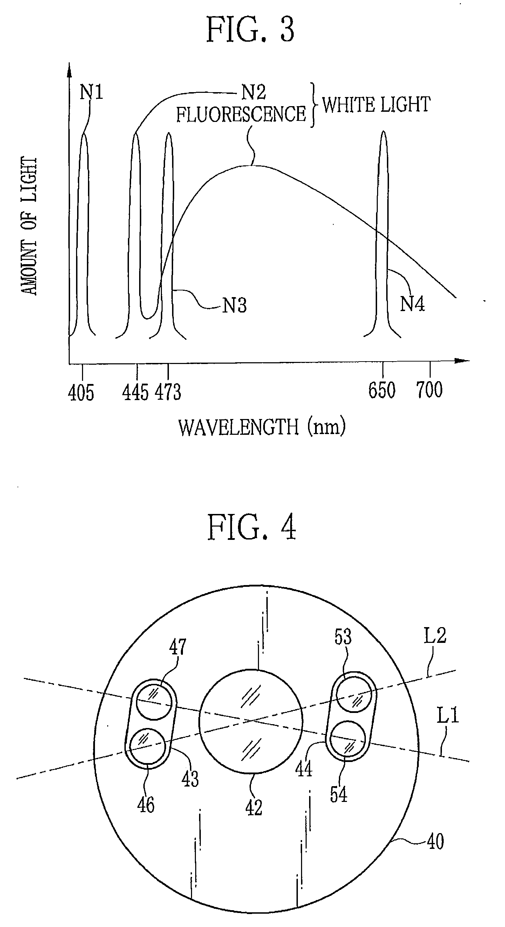

The light source device 11 is provided with four types of lasers LD1, LD2, LD3, and LD4, a light source controller 20, a combiner 21, and a splitter 22. As shown in Fig. 3, the laser LD1 generates first narrowband light N1 having a center wavelength of 405 nm. The laser LD2 generates second narrowband light N2 having a center wavelength of 445 nm. The laser LD3 generates third narrowband light N3 having a center wavelength of 473 nm. The laser LD4 generates fourth narrowband light N4 having a center wavelength of 650 nm. Of the first to fourth narrowband light N1 to N4, the second narrowband light N2 is used for exciting a phosphor 50, disposed in the distal portion 40 of the endoscope device 12, to produce white light (pseudo white light). The first to fourth narrowband light N1 to N4 is used for calculation of an oxygen saturation level of hemoglobin in blood. Note that a broad area type InGaN laser diode, an InGaNAs laser diode, a GaNAs laser diode or the like can be used for the lasers LD1, and LD2.

The first narrowband light N1 is incident on a first optical fiber 24a through a condenser lens 23a. The third narrowband light N3 is incident on a third optical fiber 24d through a condenser lens 23d. The fourth narrowband light N4 is incident on a fourth optical fiber 24e through a condenser lens 23e. When the second narrowband light N2 is used for exciting the phosphor 50 to produce the white light, the second narrowband light N2 is incident on a "2-1" optical fiber 24b through a condenser lens 23b. When the second narrowband light N2 is used for calculation of the oxygen saturation level, the second narrowband light N2 is incident on a "2-2" optical fiber 24c through light path switching mirrors 25a and 25b and a condenser lens 23c.

The light path switching mirror 25a is provided between the laser LD2 and the "2-1" optical fiber 24b. The light path switching mirror 25a is provided with a shift mechanism 30. The shift mechanism 30 shifts or moves the light path switching mirror 25a between a retracted position and an inserted position. In the retracted position, the light path switching mirror 25a is retracted from a light path of the laser LD2. In the inserted position, the light path switching mirror 25a is inserted into the light path of the laser LD2 to reflect the second narrowband light N2 to the light path switching mirror 25b. A controller 72 of the processor device 13 controls the shift mechanism 30. The light path switching mirror 25b reflects the second narrowband light N2, incident from the light path switching mirror 25a, to the condenser lens 23c.

The light source controller 20 controls the lasers LD1 to LD4 to adjust emission timing of each laser and a light quantity ratio between the lasers. The emission timing and the light quantity ratio vary between the observation modes. The combiner 21 mixes the light from the optical fibers 24a to 24e. Then, the splitter 22, being a light distributor, splits the mixed light into four paths of light.

Of the four paths of light, light guides 26 and 27 for special light transmit the light from the first, "2-2", third, and fourth optical fibers 24a, 24c, 24d, and 24e. Light guides 28 and 29 for normal light transmit the light from the "2-1" optical fiber 24b. Each of the light guides 26 to 29 is constituted of a bundle fiber that is a plurality of fibers bundled together. The light from the lasers LD1 to LD4 may be incident on the respective light guides directly without using the combiner 21 and the splitter 22.

The endoscope device 12 is composed of an electronic endoscope and is provided with a scope 32, a lighting section 33, an imaging section 34, a handling section 35, and a connector section 36. The lighting section 33 applies the four paths of light transmitted through the respective light guides 26 to 29 to the region of interest. The imaging section 34 images the region of interest. The handling section 35 is used for steering the distal portion 40 of the scope 32 and operation for the observation. The connector section 36 connects the scope 32, the light source device 11, and the processor device 13 in a detachable manner.

The scope 32 is provided with a flexible tube 38, a bending portion 39, and the distal portion 40 in this order from the handling section 35 side. When the scope 32 is inserted into the body cavity, the flexible tube 38 is flexible inside the subject. The bending portion 39 is steered by rotating an angle knob 35a disposed in the handling section 35. The bending portion 39 can be bent at any angle in any direction to direct the distal portion 40 to the region of interest.

The distal portion 40 is provided with the lighting section 33 and the imaging section 34. The imaging section 34 is provided with a capture window 42 substantially at the center of an end face of the distal portion 40. The light reflected from the region of interest is incident on the capture window 42. The lighting section 33 includes two lighting windows 43 and 44 provided on respective sides of the imaging section 34.

Two projection units 46 and 47 are disposed behind the lighting window 43. The projection unit 46 projects the first to fourth narrowband light N1 to N4 from the light guide 26 to the region of interest through a lens 48. The projection unit 47 applies the second narrowband light N2 from the light guide 28 to the phosphor 50 to produce the white light. The white light is projected to the region of interest through a lens 51. Projection units 53 and 54 are disposed behind the lighting window 44 in a manner similar to the lighting window 43. The projection unit 53 projects the first to fourth narrowband light N1 to N4 from the light guide 27 to the region of interest through the lens 48. The projection unit 54 applies the second narrowband light N2 from the light guide 29 to the phosphor 50 to project the white light to the region of interest through the lens 51.

As shown in Fig. 4, in the distal portion 40, the capture window 42 is disposed between the lighting windows 43 and 44. The four projection units 46, 47, 53, and 54 are arranged such that an alternate long and short dash line L1 between the output surfaces of the projection units 47 and 54 and an alternate long and short dash line L2 between the output surfaces of the projection units 46 and 53 cross each other at a center portion of the capture window 42. This arrangement prevents unevenness in lighting. Each of the projection units 47 and 54 projects the white light. Each of the projection units 46 and 53 projects the first to fourth narrowband light N1 to N4.

The phosphor 50 includes several kinds of fluorescent substances, for example, YAG fluorescent substances or BAM(BaMgAl₁₀O₁₇). These fluorescent substances absorb a part of the second narrowband light N2 to emit green to yellow light (fluorescence). The green to yellow light emitted from the phosphor 50 is mixed with the second narrowband light N2, passed through the phosphor 50 without being absorbed, to produce the white light (pseudo white light). A commercially available product under the product name Micro White (or MW) (registered trademark) may be used as the phosphor 50.

The white light of the present invention does not necessarily include all wavelength components of the visible light. Like the above-described pseudo white light, the white light only needs to include light in a specific wavelength range such as light of a primary color (red, green, or blue), for example. In other words, the white light may be light having wavelength components from green to red or light having wavelength components from blue to green, for example.

As shown in Fig. 2, an objective lens unit (not shown) is provided behind the capture window 42. The objective lens unit captures light (image light) reflected from the region of interest of the subject. An image sensor 60 is provided behind the objective lens unit. The image sensor 60 is a CCD (Charge Coupled Device) or a CMOS (Complementary Metal-Oxide Semiconductor), for example. The image sensor 60 receives the image light of the region of interest to produce an image thereof.

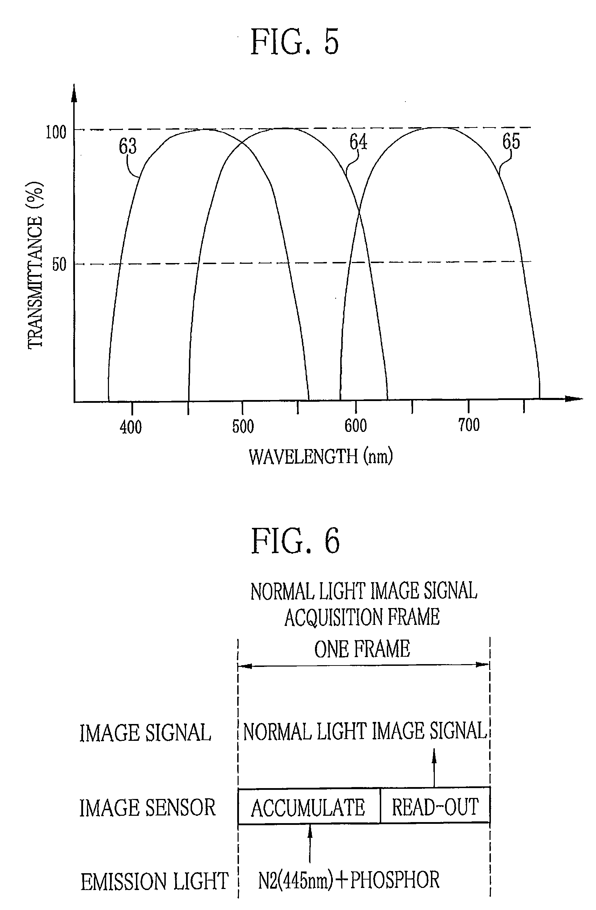

The image sensor 60 is controlled by an imaging controller 70. A light receiving surface (imaging surface) of the image sensor 60 receives the light from the objective lens unit. The image sensor 60 is a color CCD. On the light receiving surface of the image sensor 60, a pixel group composed of a plurality of pixel sets, each having an R (red) pixel, a G (green) pixel, and a B (blue) pixel, is arranged in a matrix of a predetermined pattern. Each B pixel has spectral transmittance 63, and each G pixel has spectral transmittance 64, and each R pixel has spectral transmittance 65 as shown in Fig. 5. The light received by each pixel in the image sensor 60 is photoelectrically converted into electric charge and stored. The electric charge is stored for a predetermined time. Thereafter, the electric charge stored in each pixel is read out, and the charge read-out is outputted as an image signal (analog signal) of one frame.

The image signal outputted from the image sensor 60 is inputted to an A/D converter 68 through a scope cable 67. The A/D converter 68 converts the analog image signal into a digital image signal in accordance with voltage of the analog image signal. The digital image signal includes a blue signal, a green signal, and a red signal. The blue signal is acquired by the A/D conversion of a pixel signal outputted from the B pixel. The green signal is acquired by the A/D conversion of a pixel signal outputted from the G pixel. The red signal is acquired by the A/D conversion of a pixel signal outputted from the R signal. The digital image signal is inputted to an image processor 73 of the processor device 13 through the connector section 36.

Inside the handling section 35 and the scope 32 of the endoscope device 12, various channels (not shown) are provided as is well known. The channels include, for example, an air/water channel and a forceps channel through which a sample collecting device or the like is inserted.

As shown in Fig. 2, the processor device 13 is provided with the controller 72, the image processor 73, and a storage section 74. The controller 72 is connected to the display device 14 and the input device 15. The controller 72 controls operations of the image processor 73, the light source controller 20 and the shift mechanism 30 of the light source device 11, the imaging controller 70 of the endoscope device 12, and the display device 14, according to a switching signal from the selection switch 17 of the endoscope device 12, an input signal from the input device 15, or a result of processing of the image processor 73.

The image processor 73 is provided with a normal light image processing section 80, a special image processing section 81, a distance calculating section 82, a display switching section 83, and a suspected-lesion detection section 84. The normal light image processing section 80 produces the color normal light image based on the normal light image signal. The normal light image signal is acquired by imaging the region of interest illuminated with the white light.

In the normal light image signal acquisition frame, the laser LD2 is turned on while the lasers LD1, LD3, and LD4 are turned off. The second narrowband light N2 from the laser LD2 is incident on the "2-1" optical fiber 24b. As shown in Fig. 6, by the excitation of the phosphor 50 with the second narrowband light N2, the white light is applied to the region of interest. The image sensor 60 photoelectrically converts the reflection light from the region of interest. Thus, the normal light image signal is acquired. The normal light image signal is acquired on a frame-by-frame basis.

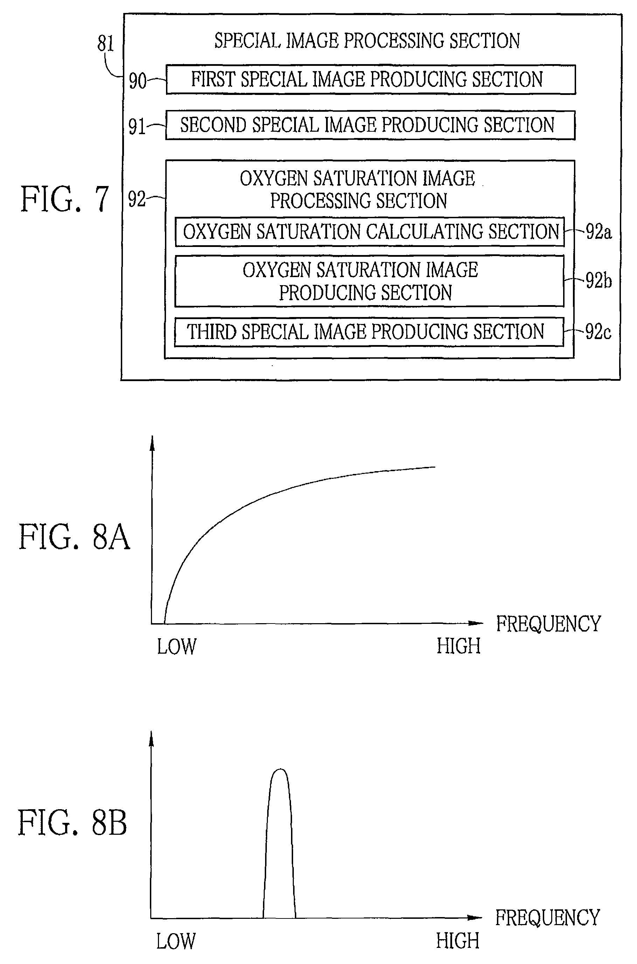

As shown in Fig. 7, the special image processing section 81 is provided with a first special image producing section 90, a second special image producing section 91, and an oxygen saturation image processing section 92. The first special image producing section 90 produces a first special image that is a composite image of the normal light image with the superficial blood vessels enhanced. The second special image producing section 91 produces a second special image in which the superficial blood vessels and the middle and deep blood vessels are enhanced. The oxygen saturation image processing section 92 calculates the oxygen saturation level of hemoglobin in blood and produces an oxygen saturation image to which colors are assigned in accordance with the oxygen saturation levels calculated. Note that the normal light image is not used as the second special image.

The first special image producing section 90 produces the normal light image (imaged with the illumination of the white light) based on the normal light image signal. The first special image producing section 90 also produces a first blue-enhanced image based on a first blue-enhanced image signal. The first blue-enhanced image signal is acquired by imaging the region of interest illuminated with the white light and the first narrowband light N1 in the blue region. The first blue-enhanced image is subjected to frequency filtering of a predetermined bandwidth. Thereby, the blood vessels and their shapes are enhanced in the image. Alternatively or in addition to the frequency filtering, the first blue-enhanced image may be subjected to color processing to display the blood vessels and mucosa in different colors and image structure processing such as sharpness enhancement and edge enhancement.

When the region of interest is in the far view, broadband frequency filtering (ranging from low frequency to high frequency, see Fig. 8A) is performed to enhance superficial blood vessels and a superficial microstructure such as a spot and a brownish area. The spot refers to a lump in which blood vessels are densely crowded. The brownish area refers to a brown-colored area in which superficial capillary vessels are densely crowded to form a lump. On the other hand, when the region of interest is in the near view, narrowband frequency filtering (at around middle frequency, see Fig. 8B) is performed to enhance broad blood vessels located deeper than the superficial layer and the shapes of the broad blood vessels.

A first special image is produced by combining the normal light image with the first blue-enhanced image subjected to the frequency filtering. The shapes of the superficial blood vessels are enhanced in the first special image. To combine the normal light image with the first blue-enhanced image, it is preferable to combine the normal light image with a B image produced based on a blue signal of the first blue-enhanced image and a G image produced based on a green signal of the first blue-enhanced image. In other words, to produce the first special image, the first blue-enhanced image with the blood vessels and their shapes enhanced is added to the normal light image with overall brightness. Thereby, the suspected lesion is surely detected in the first special image even if the light quantity is deficient, for example, when the region of interest is in the far view.

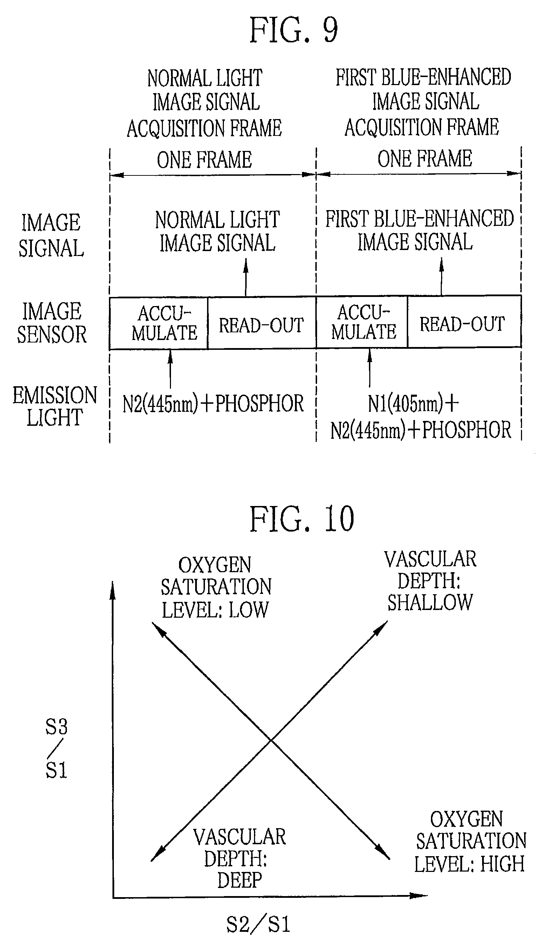

As shown in Fig. 9, the normal light image signal and the first blue-enhanced image signal, both necessary for producing the first special image, are acquired from the normal light image signal acquisition frame and the first blue-enhanced image signal acquisition frame, respectively. Namely, the image signals of two frames are acquired. In the normal light image signal acquisition frame, only the laser LD2 is turned on as described above. On the other hand, in the first blue-enhanced image signal acquisition frame, the lasers LD1 and LD2 are turned on while the lasers LD3 and LD4 are turned off. Thereby, the first and second narrowband light N1 and N2 is applied to the region of interest.

The first narrowband light N1 from the laser LD1 is applied directly to the region of interest through the first optical fiber 24a. On the other hand, the second narrowband light N2 from the laser LD2 is incident on the "2-1" optical fiber 24b. The second narrowband light N2 from the "2-1" optical fiber 24b is incident on the phosphor 50 to produce the white light by the excitation. The white light is applied to the region of interest. The image sensor 60 images the region of interest illuminated with the first narrowband light N1 and the white light. Thus, the first blue-enhanced image signal is acquired. The normal light image signal and the first blue-enhanced image signal are alternately acquired from the respective frames.

In the first blue-enhanced image signal acquisition frame, the quantity of the second narrowband light N2 (445 nm) is set greater than that of the first narrowband light N1 (405 nm), namely, the quantity of the second narrowband light N2 (445 nm) > the quantity of the first narrowband light N1(405 nm). In a light quantity ratio between the first narrowband light N1 and the white light produced by the excitation by the second narrowband light N2, the ratio of the white light is greater than that of the first narrowband light N1.

The second special image producing section 91 produces a second special image based on a second blue-enhanced image signal acquired by imaging the subject illuminated with the white light and the first narrowband light N1 in the blue region. The second blue-enhanced image signal, used for producing a second blue-enhanced image, is acquired from a second blue-enhanced image signal acquisition frame. In the second blue-enhanced image signal acquisition frame, similar to the first blue-enhanced image signal acquisition frame, the first narrowband light N1 and the white light, produced by the excitation of the phosphor 50 by the second narrowband light N2, is applied to the subject. Note that, in the second blue-enhanced image signal acquisition frame, unlike the first blue-enhanced image signal acquisition frame, the light quantity of the first narrowband light N1 is set greater than that of the second narrowband light N2, namely, the quantity of first narrowband light N1 (405 nm) > the quantity of the second narrowband light N2 (445 nm). By imaging a reflected image of the subject, the second blue-enhanced image signal is acquired.

A blue signal of the second blue-enhanced image signal is assigned to a B channel and a G channel of a display signal. A green signal of the second blue-enhanced image signal is assigned to an R channel of the display signal. Thereby, the second special image is produced. The second special image is substantially the same as an image produced using NBI (narrowband imaging) in which the blue narrowband signal having a blue narrowband component of 415 nm is assigned to B and G channels of the display signal and a green narrowband signal having a green narrowband component of 540 nm is assigned to the R channel of the display signal. The absorbance of hemoglobin in blood is high both at the wavelengths 415 nm and 540 nm. Accordingly, broad blood vessels in the middle and deep layer and their shapes are displayed clearly in the second special image in addition to the superficial capillary vessels and their shapes.

As shown in Fig. 7, the oxygen saturation image processing section 92 is provided with an oxygen saturation level calculation section 92a, an oxygen saturation image producing section 92b, and a third special image producing section 92c. The oxygen saturation level calculation section 92a calculates the oxygen saturation level of hemoglobin in blood. The oxygen saturation image producing section 92b produces the oxygen saturation image based on the oxygen saturation level calculated. The third special image producing section 92c produces a third special image. To be more specific, the third special image is produced by reflecting information of the oxygen saturation level in the first special image. The oxygen saturation level calculating section 92a uses a first oxygen saturation signal S1, a second oxygen saturation signal S2, and a third oxygen saturation signal S3 to obtain or calculate the oxygen saturation level of the blood vessel in the superficial to middle and deep layer. The imaging using the first narrowband light N1 provides the first oxygen saturation signal S1. The imaging using the second narrowband light N2 provides the second oxygen saturation signal S2. The imaging using the third narrowband light N3 provides the third oxygen saturation signal S3. In addition to the first and third oxygen saturation signals S1 and S3, a fourth oxygen saturation signal S4 is used to obtain the oxygen saturation level of the blood vessel in the middle and deep layer. The imaging using the fourth narrowband light N4 provides the fourth oxygen saturation signal S4.

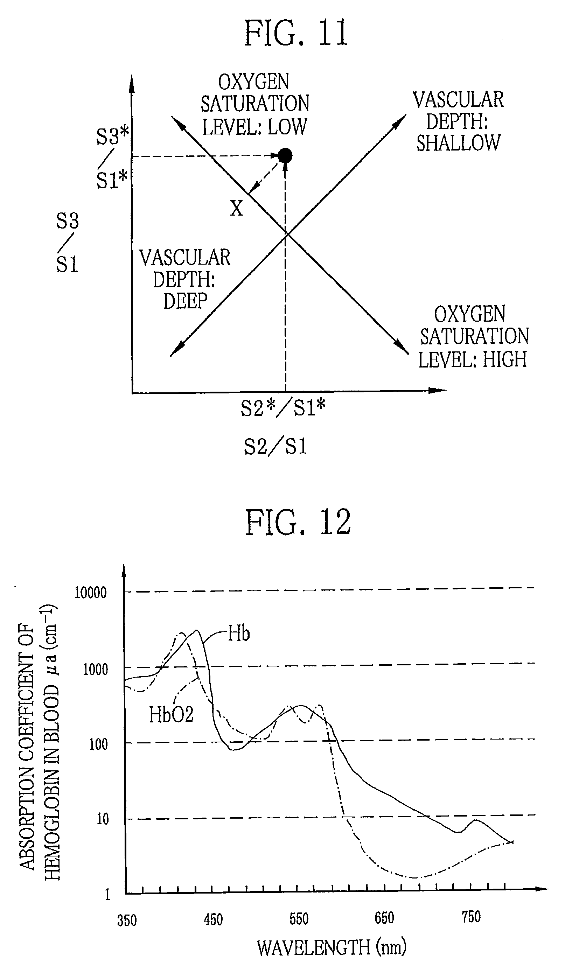

To calculate the oxygen saturation level of the blood vessel in the superficial to middle and deep layer, first, the signal ratio S2/S1 between the second oxygen saturation signal S2 and the first oxygen saturation signal S1 and the signal ratio S3/S1 between the third oxygen saturation signal S3 and the first oxygen saturation signal S1 are obtained. Next, as shown in Fig. 10, the oxygen saturation level in each pixel is obtained using the correlation among the signal ratios S2/S1 and S3/S1, the vascular depth and the oxygen saturation level. The correlation is obtained from the past diagnoses or the like and stored in the storage section 74 in advance. For example, as shown in Fig. 11, when the signal ratios are S2*/S1* and S3*/S1*, the oxygen saturation level corresponding to the signal ratios is "X(%)".

Note that, as shown in Fig. 12, the signal "S2" (obtained from the light at the 445 nm wavelength range) of the signal ratio S2/S1 and the signal "S3" (obtained from the light at the 473 nm wavelength range) of the signal ratio S3/S1 are those obtained from the light in a wavelength range in which oxyhemoglobin (HbO₂) and deoxyhemoglobin (Hb) are different in absorbance. Accordingly, the absorbance varies with the oxygen saturation level in blood, which in result changes the signal values. In other words, the signal ratios S2/S1 and S3/S1 include information of the oxygen saturation level.

However, the signal "S2" (obtained from the light at the 445 nm wavelength range) and the signal "S3" (obtained from the light at the 473 nm wavelength range) are different in light penetration depth. Accordingly, the signal ratios S2/S1 and S3/S1 include information of vascular depth in addition to the information of the oxygen saturation level. For this reason, the signal ratios S2/S1 and S3/S1 themselves may not represent the information of the oxygen saturation level accurately. With the use of the correlation shown in Fig. 10, the accurate information of the oxygen saturation level is obtained by separating the information of the oxygen saturation level from the information of the vascular depth, and extracting only the information of the oxygen saturation level.

To calculate or obtain the oxygen saturation level of the blood vessel in the middle and deep layer, the signal ratio S3/S1 between the third oxygen saturation signal S3 and the first oxygen saturation signal S1 and the signal ratio S4/S1 between the fourth oxygen saturation signal S4 and the first oxygen saturation signal S1 are obtained in a manner similar to the above. With the use of the correlation among the signal ratios S3/S1, S4/S1, and the oxygen saturation level, obtained from the past diagnoses, the oxygen saturation level in each pixel is obtained.

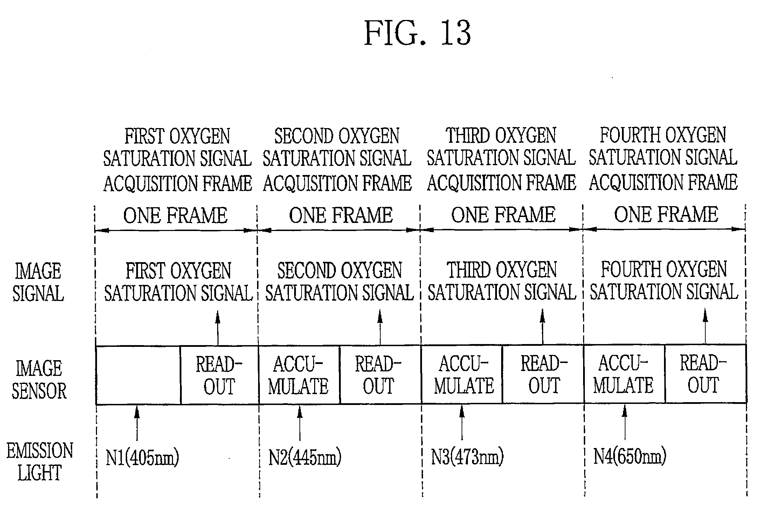

Note that, as shown in Fig. 13, the first to fourth oxygen saturation signals are obtained from four frames, that is, first to fourth oxygen saturation signal acquisition frames, respectively. In the first oxygen saturation signal acquisition frame, the laser LD1 is turned on while the lasers LD2, LD3, and LD4 are turned off. The first narrowband light N1 from the laser LD1 is incident on the first optical fiber 24a. The first narrowband light N1 is applied to the region of interest, which is imaged by the image sensor 60. Thereby, the first oxygen saturation signal is acquired.

In the second oxygen saturation signal acquisition frame, the laser LD2 is turned on while the lasers LD1, LD3, and LD4 are turned off. The second narrowband light N2 from the laser LD2 is applied to the region of interest through the "2-2" optical fiber 24c. The image sensor 60 images the region of interest illuminated with the second narrowband light N2. Thus, the second oxygen saturation signal is acquired.

In a third oxygen saturation signal acquisition frame, only the laser LD3 is turned on. The third narrowband light N3 from the laser LD3 is applied to the region of interest through the third optical fiber 24d. The image sensor 60 images the region of interest illuminated with the third narrowband light N3. Thereby, the third oxygen saturation signal is acquired. In the fourth oxygen saturation signal acquisition frame, only the laser LD4 is turned on. The fourth narrowband light N4 from the laser LD4 is applied to the region of interest through the fourth optical fiber 24e. The image sensor 60 images the region of interest illuminated with the fourth narrowband light N4. Thereby, the fourth oxygen saturation signal is acquired.

The oxygen saturation image producing section 92b produces an oxygen saturation image, that is, an image of the oxygen saturation level(s) of blood vessel(s), strictly speaking, the oxygen saturation level(s) of blood in blood vessel(s), calculated by the oxygen saturation level calculating section 92a. For example, to produce a pseudo color image, the oxygen saturation image producing section 92b assigns different colors to the blood vessels in the image according to their oxygen saturation levels. Alternatively, the oxygen saturation image producing section 92b may produce a monochrome image in which the oxygen saturation levels are depicted in various shades.

In the third special image producing section 92c, the oxygen saturation level, calculated by the oxygen saturation level calculating section 92a, is reflected in the first special image in which the shapes of the superficial blood vessels are enhanced. Thus, the third special image is produced. To be more specific, when the oxygen saturation level of an area exceeds a predetermined range (for example, when the predetermined range is 0% to 60% and the oxygen saturation level exceeds 60%), the information of the oxygen saturation level is not reflected in the first special image. On the other hand, when the oxygen saturation level of an area is in a hypoxic condition or a state of oxygen deficiency within the predetermined range, the information of the oxygen saturation level is reflected in the first special image such that the image is depicted in pseudo colors, for example. Thereby, the third special image offers information of the surface condition, for example, projections and depressions on the surface of the region of interest shown in the normal light image, in addition to the oxygen saturation levels of the blood vessels. This improves the diagnostic performance. The lower limit of the above-described predetermined range is "0%" by way of example. The lower limit may be greater than "0%"

To produce the oxygen saturation image in the oxygen saturation image producing section 92b or when the oxygen saturation level (the oxygen saturation image) is reflected in the first special image in the third special image producing section 92c, it is preferable to produce the oxygen saturation image from an average of the oxygen saturation level calculated using the first to third oxygen saturation signals S1 to S3 and the oxygen saturation level calculated using the first, third, and fourth oxygen saturation signals S1, S3, and S4, or one of the oxygen saturation levels.

The distance calculating section 82 calculates or obtains an observation distance (see Fig. 14) between the distal portion 40 and the region of interest "R" based on the images produced in the normal light image processing section 80 and the special image processing section 81. The distance calculating section 82 calculates or obtains an average luminance value based on the images. The observation distance is determined based on the average luminance value. The greater the average luminance value, the shorter the observation distance. For example, when the average luminance value is high, it is considered that the distal portion 40 is close to the region of interest R, so that the quantity of light returning to the distal portion 40 increases. Accordingly, it is judged that the region of interest R is in the near view. On the other hand, when the average luminance value is low, it is considered that the distal portion 40 is away from the region of interest R, so that the quantity of light returning to the distal portion 40 decreases. Accordingly, it is judged that the region of interest R is in the far view.

In the automatic exposure control, an appropriate exposure amount is automatically set in accordance with the luminance value or the like. Generally, when the region of interest R is in the near view and the light quantity is high, the exposure amount is set low. On the contrary, when the region of interest R is in the far view and the light quantity is lower than the appropriate value, the exposure amount is set high. The observation distance is estimated or determined based on the exposure amount.

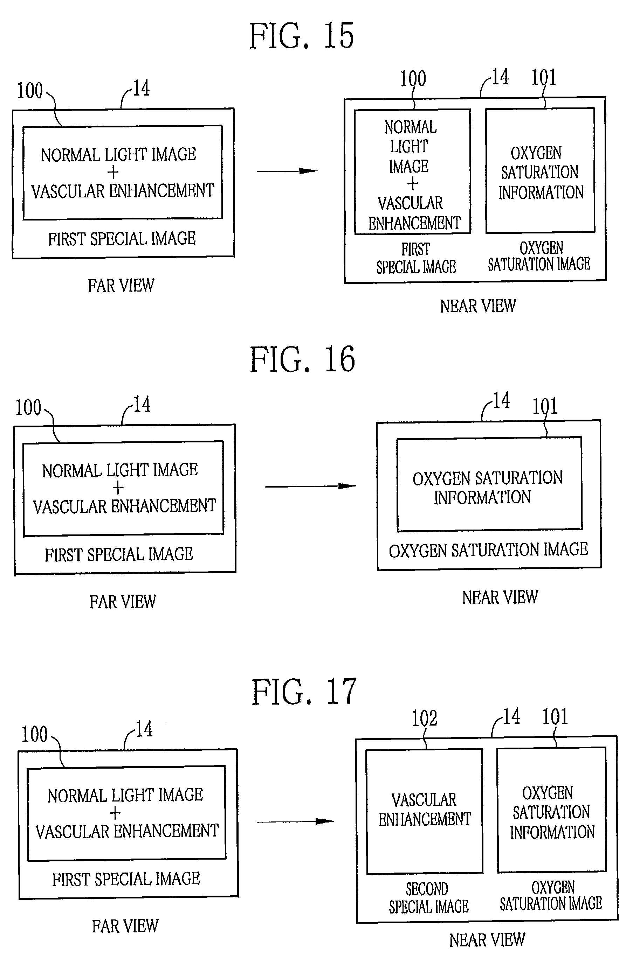

In the "1-1" to "1-5" special observation modes, the display switching section 83 switches the content of the display image (s) based on the observation distance. In the "1-1" special observation mode, as shown in Fig. 15, a first special image 100 is displayed on the display device 14 when the region of interest R is in the far view. The first special image 100 is the composite image of the normal light image with the surface blood vessels enhanced. Thereby, the screening of the region of interest in the far view surely detects suspected lesions such as the spots and the brownish areas. When the observation distance measured by the distance calculating section 82 is less than the predetermined value, namely, when the region of interest R is in the near view, an oxygen saturation image 101 is displayed in addition to the first special image 100 on the display device 14. The use of the oxygen saturation image 101 improves accuracy in distinguishing the cancer from other lesions in differential diagnosis of cancer performed when the region of interest is in the near view.

In the "1-2" special observation mode, as shown in Fig. 16, the first special image 100 is displayed on the display device 14 when the region of interest R is in the far view. When the observation distance calculated by the distance calculating section 82 is less than the predetermined value, namely, when the region of interest R is in the near view, only the oxygen saturation image 101 is displayed instead of the first special image 100 on the display device 14.

In the "1-3" special observation mode, as shown in Fig. 17, the first special image 100 is displayed on the display device 14 when the region of interest R is in the far view. When the observation distance calculated by the distance calculating section 82 is less than the predetermined value, namely, when the region of interest R is in the near view, two types of images, a second special image 102 and the oxygen saturation image 101 are displayed instead of the first special image 100 on the display device 14. The second special image 102 is substantially the same as the narrowband light image produced from the blue narrowband light with the center wavelength of 415 nm and the green narrowband light with the center wavelength of 540 nm.

Of the biological information relevant to cancer, a pattern (structure) of the blood vessel(s) and the shape of the uneven surface (for example, the projections and depressions) are apparent in the second special image 102. The oxygen condition (the oxygen saturation level) of hemoglobin in blood is apparent in the oxygen saturation image 101. Accordingly, by using the two types of images, the second special image 102 and the oxygen saturation image 101, in the diagnosis, the cancer is surely distinguished from other lesions. Note that, in displaying the second special image 102 and the oxygen saturation image 101, it is preferable to set the update timing of the oxygen saturation image 101 faster than that of the second special image 102 to give priority to moving picture performance of the oxygen saturation image 101.

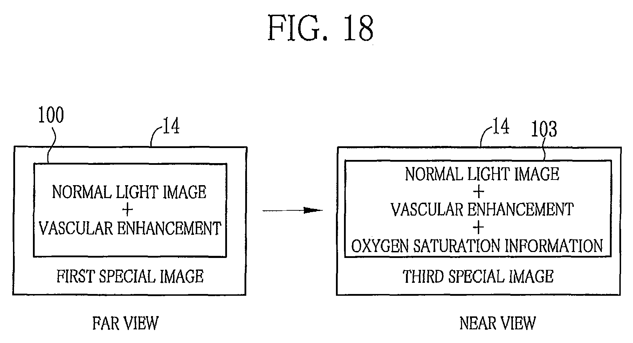

In the "1-4" special observation mode, as shown in Fig. 18, the first special image 100 is displayed on the display device 14 when the region of interest R is in the far view. When the observation distance calculated by the distance calculating section 82 is less than the predetermined value, namely, when the region of interest R is in the near view, a third special image 103 is displayed instead of the first special image 100 on the display device 14. The third special image 103 is the composite image of the first special image in which the information of the oxygen saturation level is reflected.

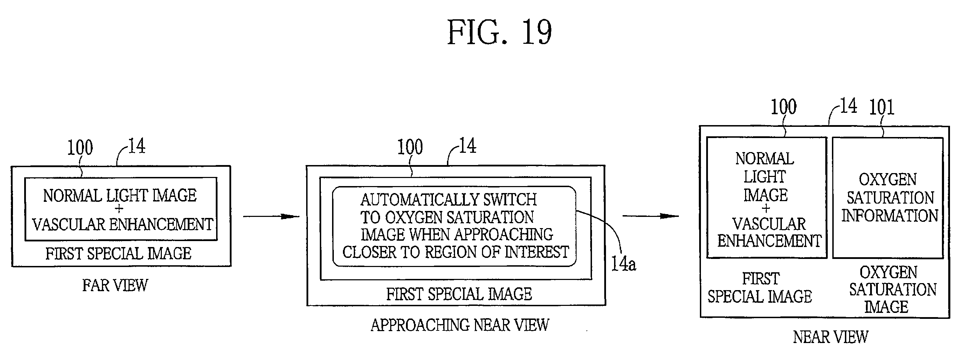

In the "1-5" special observation mode, as shown in Fig. 19, the first special image 100 is displayed on the display device 14 when the region of interest R is in the far view. When the observation distance calculated by the distance calculating section 82 is decreased to the predetermined value, meaning that the region of interest R is in the near view, a message ("automatically switch to the oxygen saturation image when approaching closer to the region of interest R") 14a is displayed on the display device 14. Then, when the region of interest R is in the near view, the oxygen saturation image 101 is displayed in addition to the first special image 100 on the display device 14 in a manner similar to the "1-1" observation mode. Note that when the region of interest R is in the near view, the image (s) of the region of interest in the near view displayed in the "1-2" to "1-5" special observation modes may be displayed on the display device 14.

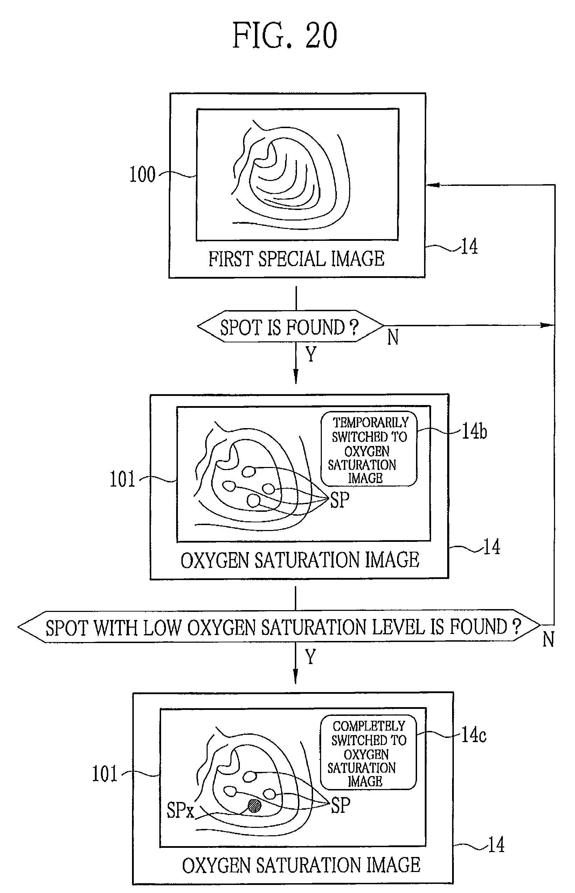

In the second special observation mode, the suspected-lesion detection section 84 detects the suspected lesion in the far view. To be more specific, as shown in Fig. 20, when the region of interest R is in the far view, the first special image 100 is obtained and displayed on the display device 14. The suspected-lesion detection section 84 detects a spot SP, being one type of the suspected lesions, at regular intervals. The spot SP is detected using image processing, for example, pattern matching.

When the suspected-lesion detection section 84 detects a spot SP larger than predetermined size or two or more spots SP each smaller than the predetermined size, the oxygen saturation image 101 is obtained temporarily. Then, the suspected-lesion detection section 84 detects whether the oxygen saturation level of each spot SP in the oxygen saturation image 101 is in the hypoxic condition within a predetermined range. During the detection, the temporarily obtained oxygen saturation level (the oxygen saturation image 101) is displayed on the display device 14, together with a message 14b notifying that the display is currently switched to the oxygen saturation image temporarily.

As a result of the detection, when there is no spot SPx in the hypoxic condition, the display of the oxygen saturation image 101 is discontinued and the first special image 100 is displayed again. On the other hand, when one or more spots SPx are in the hypoxic condition, the display device 14 keeps displaying the oxygen saturation image 101 together with a message 14c notifying that the display image is completely switched to the oxygen saturation image.

In the above embodiments, the oxygen saturation level is calculated using the first to fourth oxygen saturation signals. It is also possible to calculate the oxygen saturation level using two types of signals: the third oxygen saturation signal acquired by imaging the region of interest illuminated with the third narrowband light N3 (with the center wavelength of 473 nm) and the normal light image signal. Note that the third oxygen saturation signal and the normal light image signal are acquired from the respective different frames.

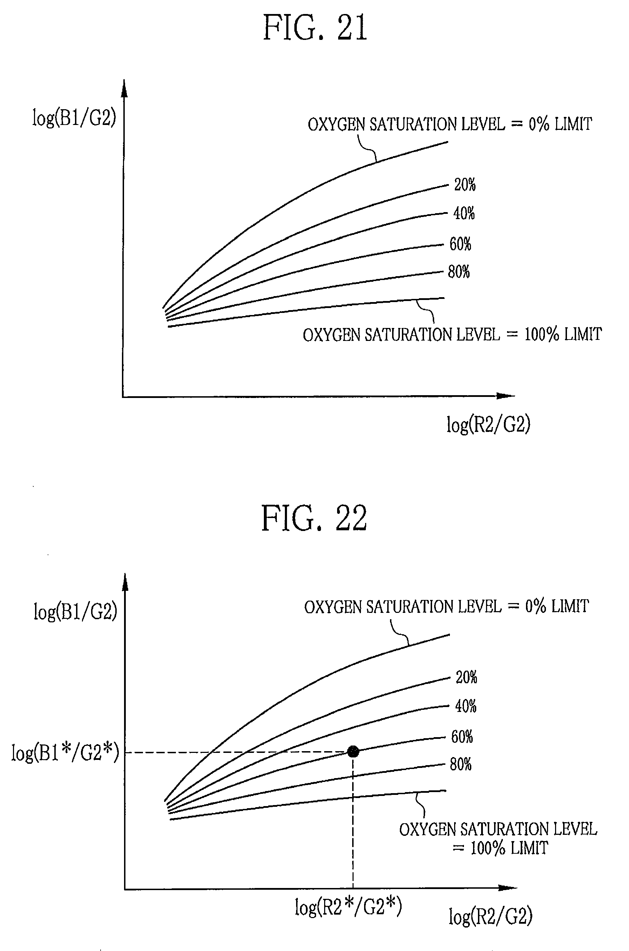

In this case, first, a signal ratio B1/G2, that is, a signal ratio between the blue signal B1 of the third oxygen saturation signal and the green signal G2 of the normal light image signal, and a signal ratio R2/G2, that is, a signal ratio between the red signal R2 of the normal light image signal and the green signal G2 of the normal light image signal are calculated or obtained. Next, as shown in Fig. 21, the oxygen saturation level in each pixel is calculated or obtained with the use of the correlation among the signal ratios B1/G2 and R2/G2, the blood volume, and the oxygen saturation level, obtained from the past diagnoses. The correlation is stored in the storage section 74 in advance. For example, as shown in Fig. 22, when the signal ratios are B1*/G2* and R2*/G2*, the oxygen saturation level corresponding to the signal ratios is 60 (%).

In the above embodiments, the lasers are used for illuminating the region of interest. Instead, the region of interest may be illuminated using a frame sequential method. In the frame sequential method, a broadband light source, for example, a xenon lamp for emitting the white light and a rotating filter are used. The rotating filter has two or more bandpass filters provided along a circumferential direction. Each bandpass filter passes light in a specific wavelength range used in the corresponding observation mode.

Note that, in the above embodiments, the display image is automatically switched from an image for screening to an image for detailed diagnosis when the observation distance is less than a predetermined value, namely, when the region of interest is in the near view. Additionally, the display image may be automatically switched from the image for the detailed diagnosis to the image for the screening when the region of interest is in the far view.

Various changes and modifications are possible within the scope of the claims and may be understood to be within the present invention.

## Claims

1. An endoscope system (10) for observing a region of interest in a body cavity, the endoscope system **characterized by** comprising:
a lighting section (33) for applying illumination light, selected from two or more types of illumination light, to a region of interest;
an imaging section (60) for imaging the region of interest illuminated with the illumination light selected;
an image processor (73) for producing an observation image based on an image signal acquired from the imaging section, the observation image corresponding to the illumination light selected;
a suspected-lesion detection section (84) for detecting a suspected lesion in the observation image;
an oxygen saturation calculation section (92a) for obtaining an oxygen saturation level of a blood vessel in the observation image;
a judging section (84) for judging whether the oxygen saturation level of the suspected lesion is in a hypoxic condition within a predetermined range; and
a display section (14) for displaying the observation image; **characterized in that** the lighting section selectively generates one of white light, mixed light in which the white light is mixed with blue narrowband light at a predetermined ratio, and two or more types of narrowband light each having an absorption coefficient varying with the oxygen saturation level, as the illumination light, and **in that** the image processor includes a special image processing section (81) for combining a normal light image with a blue-enhanced image to produce a special image in which the blood vessel is enhanced, and the normal light image is produced based on the image signal outputted from the imaging section during illumination with the white light, and the blue-enhanced image is produced based on the image signal outputted from the imaging section during illumination with the mixed light.

2. The endoscope system of claim 1, wherein the lighting section alternately generates the white light and the mixed light in respective frames, and the imaging section alternately outputs the image signal of the normal light image of one frame and the image signal of the blue-enhanced image of one frame.

3. The endoscope system of claim 2, wherein the special image processing section performs frequency filtering of a bandwidth ranging from a low frequency to a high frequency to the blue-enhanced image before combining the normal light image with the blue-enhanced image.

4. The endoscope system of claim 1, wherein the oxygen saturation calculating section acquires two or more oxygen saturation signals from the image signals outputted from the imaging section during sequential illumination with the respective narrowband light, and the image signals correspond to the respective narrowband light, and the oxygen saturation level is obtained from magnitude of a ratio between the oxygen saturation signals.

5. The endoscope system of claim 4, wherein the image processor further includes an oxygen saturation image processing section (92b) for producing an oxygen saturation image that is an image of the blood vessel represented by the oxygen saturation level.

6. The endoscope system of claim 5, wherein the blood vessel in the oxygen saturation image is colored in accordance with the oxygen saturation level.

7. The endoscope system of claim 5, **characterized by** further including:
a display controller (72) for allowing the display section to display the special image before the suspected lesion is detected, and to display the oxygen saturation image when the suspected lesion is judged to be in the hypoxic condition.

8. The endoscope system of claim 7, wherein the display controller allows the display section to temporarily display the oxygen saturation image between after the suspected lesion is detected and before the suspected lesion is judged to be in the hypoxic condition.

9. The endoscope system of claim 7, wherein before the oxygen saturation image is displayed on the display section, the display controller displays a message on the display section, and the message notifies that the oxygen saturation image will be displayed on the display section.

10. A method for assisting in diagnostic endoscopy **characterized by** comprising the steps of:
applying white light to a region of interest in a body cavity;
producing a normal light image based on an image signal outputted from an imaging section (60) during illumination with the white light;
applying mixed light of the white light and blue narrowband light to the region of interest, the white light and the blue narrowband light being mixed at a predetermined ratio;
producing a blue-enhanced image based on an image signal outputted from the imaging section during illumination with the mixed light;
combining the normal light image with the blue-enhanced image to produce a special image in which a blood vessel is enhanced;
detecting a suspected lesion in the special image;
applying two or more types of narrowband light sequentially to the region of interest, an absorption coefficient of the each narrowband light varying with an oxygen saturation level;
acquiring two or more oxygen saturation signals from image signals, corresponding to the respective narrowband light, outputted from the imaging section during sequential illumination with the narrowband light;
obtaining an oxygen saturation level of blood in the blood vessel from magnitude of a ratio between the oxygen saturation signals;
producing an oxygen saturation image that is an image of the blood vessel represented by the oxygen saturation level;
judging whether the oxygen saturation level in the suspected lesion is in a hypoxic condition within a predetermined range; and
displaying the special image on a display section (14) before the suspected lesion is detected, and displaying the oxygen saturation image on the display section when the suspected lesion is judged to be in the hypoxic condition.

11. The method of claim 10, wherein the oxygen saturation image is temporarily displayed on the display section between after the suspected lesion is detected and before the suspected lesion is judged to be in the hypoxic condition.

## Patentansprüche

1. Endoskopsystem (10) zum Betrachten einer interessierenden Zone innerhalb eines Körperhohlraums, **gekennzeichnet durch**:
einen Beleuchtungsabschnitt (33) zum Aufbringen von aus zwei oder mehr Typen von Beleuchtungslicht ausgewähltem Beleuchtungslicht auf eine interessierende Zone;
einen Bildgebungsabschnitt (60) zum Abbilden der interessierenden Zone, die mit dem ausgewählten Beleuchtungslicht beleuchtet wird;
einen Bildprozessor (73) zum Erzeugen eines Betrachtungsbilds basierend auf einem von dem Bildgebungsabschnitt erfassten Bildsignal, wobei das Betrachtungsbild dem ausgewählten Beleuchtungslicht entspricht;
einen Verdachtsläsion-Nachweisabschnitt (84) zum Nachweisen einer mutmaßlichen Läsion in dem Betrachtungsbild;
einen Sauerstoffsättigungs-Berechnungsabschnitt (92a) zum Ermitteln eines Sauerstoffsättigungswerts eines Blutgefäßes in dem Betrachtungsbild;
einen Beurteilungsabschnitt (84) zum Beurteilen, ob der Sauerstoffsättigungswert der mutmaßlichen Läsion sich innerhalb eines vorbestimmten Bereichs in einem hypoxischen Zustand befindet; und
einen Anzeigeabschnitt (14) zum Anzeigen des Betrachtungsbilds, **dadurch** gekennzeichnet, dass der Beleuchtungsabschnitt selektiv weißes Licht, gemischtes Licht, in welchem das weiße Licht mit blauem schmalbandigem Licht in einem vorbestimmten Verhältnis gemischt ist, oder zwei oder mehr Typen schmalbandigen Lichts mit jeweils einem Absorptionskoeffizienten, der mit dem Sauerstoffsättigungswert schwankt, als das Beleuchtungslicht erzeugt, und dass der Bildprozessor einen Spezialbild-Verarbeitungsabschnitt (61) zum Kombinieren eines Normallichtbilds mit einem blauverstärkten Bild kombiniert, um ein Spezialbild zu erzeugen, in welchem das Blutgefäß hervorgehoben ist, und das Normallichtbild basierend auf dem Bildsignal erzeugt wird, welches von dem Bildgebungsabschnitt während der Beleuchtung mit dem weißen Licht ausgegeben wird, und das blauverstärkte Bild basierend auf dem Bildsignal erzeugt wird, welches von dem Bildgebungsabschnitt während der Beleuchtung mit dem gemischten Licht ausgegeben wird.

2. Endoskopsystem nach Anspruch 1, bei dem der Beleuchtungsabschnitt abwechselnd das weiße Licht und das gemischte Licht in zugehörigen Vollbildern erzeugt, und der Bildgebungsabschnitt alternierend das Bildsignal des Normallichtbilds eines Vollbilds und das Bildsignal des blauverstärkten Bilds eines Vollbilds ausgibt.

3. Endoskopsystem nach Anspruch 2, bei dem der Spezialbild-Verarbeitungsabschnitt eine Frequenzfilterung einer Bandbreite von einer niedrigen Frequenz bis zu einer hohen Frequenz an dem blauverstärkten Bild ausführt, bevor das Normallichtbild mit dem blauverstärkten Bild kombiniert wird.

4. Endoskopsystem nach Anspruch 1, bei dem der Sauerstoffsättigungs-Berechnungsabschnitt zwei oder mehr Sauerstoffsättigungssignale aus den von dem Bildgebungsabschnitt während sequenzieller Beleuchtung mit dem jeweiligen schmalbandigen Licht ausgegebenen Bildsignalen erfasst, und die Bildsignale dem jeweiligen schmalbandigen Licht entsprechen, und der Sauerstoffsättigungswert erhalten wird aus dem Betrag eines Verhältnisses zwischen den Sauerstoffsättigungssignalen.

5. Endoskopsystem nach Anspruch 4, bei dem der Bildprozessor weiterhin einen Sauerstoffsättigungsbild-Verarbeitungsabschnitt (92b) zum Erzeugen eines Sauerstoffsättigungsbilds enthält, bei dem es sich um ein Bild des durch den Sauerstoffsättigungswert repräsentierten Blutgefäßes handelt.

6. Endoskopsystem nach Anspruch 5, bei dem das Blutgefäß in dem Sauerstoffsättigungsbild nach Maßgabe des Sauerstoffsättigungspegels eingefärbt ist.

7. Endoskopsystem nach Anspruch 5, weiterhin **gekennzeichnet durch**
eine Anzeigesteuerung (72), die es dem Anzeigeabschnitt ermöglicht, das Spezialbild vor dem Nachweis der mutmaßlichen Läsion anzuzeigen, und das Sauerstoffsättigungsbild anzuzeigen, wenn die mutmaßliche Läsion dahingehend beurteilt wird, dass sie sich in dem hypoxischen Zustand befindet.

8. Endoskopsystem nach Anspruch 7, bei dem die Anzeigesteuerung dem Anzeigeabschnitt ermöglicht, vorübergehend das Sauerstoffsättigungsbild nach dem Nachweisen der mutmaßlichen Läsion und vor der Beurteilung der mutmaßlichen Läsion als in dem hypoxischen Zustand befindlich anzuzeigen.

9. Endoskopsystem nach Anspruch 7, bei dem vor dem Anzeigen des Sauerstoffsättigungsbilds auf dem Anzeigeabschnitt die Anzeigesteuerung eine Nachricht auf dem Anzeigeabschnitt anzeigt, und die Nachricht bedeutet, dass das Sauerstoffsättigungsbild auf dem Anzeigeabschnitt angezeigt wird.

10. Verfahren zum Unterstützen einer diagnostischen Endoskopie, **gekennzeichnet durch** folgende Schritte:
Aufbringen von weißem Licht auf eine interessierende Zone in einen Körperhohlraum;
Erzeugen eines Normallichtbilds basierend auf einem Bildsignal, welches von einem Bildgebungsabschnitt (60) während der Beleuchtung mit dem weißen Licht ausgegeben wird;
Aufbringen von gemischtem Licht aus weißem Licht und blauem, schmalbandigem Licht auf die interessierende Zone, wobei das weiße Licht und das blaue, schmalbandige Licht in einem vorbestimmten Verhältnis gemischt sind;
Erzeugen eines blauverstärkten Bilds basierend auf einem Bildsignal, das von dem Bildgebungsabschnitt während der Beleuchtung mit dem gemischten Licht ausgegeben wird;
Kombinieren des Normallichtbilds mit dem blauverstärkten Bild, um ein Spezialbild zu erzeugen, in welchem ein Blutgefäß hervorgehoben ist;
Nachweisen einer mutmaßlichen Läsion in dem Spezialbild;
Aufbringen von zwei oder mehr Typen schmalbandigen Lichts auf die interessierende Zone in sequenzieller Weise, wobei ein Absorptionskoeffizient für jedes schmalbandige Licht mit einem Sauerstoffsättigungswert schwankt;
Erfassen von zwei oder mehr Sauerstoffsättigungssignalen aus den Bildsignalen, entsprechend dem jeweiligen schmalbandigen Licht, das von dem Bildgebungsabschnitt während der sequenziellen Beleuchtung mit dem schmalbandigen Licht ausgegeben wird;
Ermitteln eines Sauerstoffsättigungswerts von Blut in dem Blutgefäß anhand des Betrags eines Verhältnisses zwischen den Sauerstoffsättigungssignalen;
Erzeugen eines Sauerstoffsättigungsbilds, bei dem es sich um ein Bild des durch den Sauerstoffsättigungswert repräsentierten Blutgefäßes handelt;
Beurteilen, ob der Sauerstoffsättigungswert in der mutmaßlichen Läsion sich in einem hypoxischen Zustand innerhalb eines vorbestimmten Bereichs befindet; und
Anzeigen des Spezialbilds auf einem Anzeigeabschnitt (14) vor Nachweis der mutmaßlichen Läsion, und Anzeigen des Sauerstoffsättigungsbilds auf dem Anzeigeabschnitt, wenn von der mutmaßlichen Läsion beurteilt wird, dass sie sich in dem hypoxischen Zustand befindet.

11. Verfahren nach Anspruch 10, bei dem das Sauerstoffsättigungsbild vorübergehend auf dem Anzeigeabschnitt nach dem Nachweis der mutmaßlichen Läsion und vor der Beurteilung, dass sich die mutmaßliche Läsion in dem hypoxischen Zustand befindet, angezeigt wird.

## Revendications

1. Système d'endoscope (10) destiné à observer une région d'intérêt dans une cavité corporelle, le système d'endoscope étant **caractérisé en ce qu'**il comprend :
une partie d'éclairage (33) destinée à appliquer une lumière d'éclairement, sélectionnée parmi un ou deux types de lumière d'éclairement, à une région d'intérêt ;
une partie d'imagerie (60) destinée à imager la région d'intérêt éclairée avec la lumière d'éclairement sélectionnée ;
un processeur d'image (73) destiné à produire une image d'observation sur la base d'un signal image acquis à partir de la partie d'imagerie, l'image d'observation correspondant à l'image d'éclairement sélectionnée ;
une partie de détection de lésion suspectée (84) destinée à détecter une lésion suspectée dans l'image d'observation ;
une partie de calcul de saturation en oxygène (92a) destinée à obtenir un niveau de saturation en oxygène d'un vaisseau sanguin dans l'image d'observation ;
une partie de jugement (84) destinée à juger si le niveau de saturation en oxygène de la lésion suspectée est dans un état hypoxique dans les limites d'une plage prédéterminée, et
une partie d'affichage (14) destinée à afficher l'image d'observation,
**caractérisé en ce que**
la partie d'éclairage génère de manière sélective une parmi une lumière blanche, une lumière mixte, où la lumière blanche est mélangée à une lumière de bande étroite bleue selon un rapport prédéterminé, et deux types ou plus de lumière de bande étroite, chacun présentant un coefficient d'absorption variant avec le niveau de saturation en oxygène, comme la lumière d'éclairement, et **en ce que** le processeur d'image inclut une partie de traitement d'image spéciale (81) destinée à combiner une image de lumière normale avec une image présentant un bleu de mise en valeur afin de produire une image spéciale dans laquelle le vaisseau sanguin est mis en valeur, et l'image de lumière normale est produite sur la base du signal image émis à partir de la partie d'imagerie durant l'éclairement avec la lumière blanche, et l'image présentant le bleu de mise en valeur est produite sur la base du signal image émis à partir de la partie d'imagerie durant l'éclairement avec la lumière mixte.

2. Système d'endoscope selon la revendication 1, dans lequel la partie d'éclairage génère par alternance la lumière blanche et la lumière mixte dans des trames respectives, et la partie d'imagerie produit par alternance le signal image de l'image de lumière normale d'une trame et le signal image de l'image présentant le bleu de mise en valeur d'une trame.

3. Système d'endoscope selon la revendication 2, dans lequel la partie de traitement d'image spéciale exécute un filtrage de fréquence d'une largeur de bande s'étendant dans une plage allant d'une basse fréquence à une haute fréquence sur l'image présentant le bleu de mise en valeur avant combinaison de l'image de lumière normale avec l'image présentant le bleu de mise en valeur.

4. Système d'endoscope selon la revendication 1, dans lequel la partie de calcul de saturation en oxygène acquiert deux signaux de saturation en oxygène ou plus, à partir de signaux image, émis à partir de la partie d'imagerie durant l'éclairement séquentiel avec la lumière de bande étroite respective, et les signaux image correspondent à la lumière de bande étroite respective, et le niveau de saturation en oxygène est obtenu à partir de l'amplitude d'un rapport entre les signaux de saturation en oxygène.

5. Système d'endoscope selon la revendication 4, dans lequel le processeur d'image inclut en outre une partie de traitement d'image de saturation en oxygène (92b) destinée à produire une image de saturation en oxygène qui est une image du vaisseau sanguin représenté par le niveau de saturation en oxygène.

6. Système d'endoscope selon la revendication 5, dans lequel le vaisseau sanguin dans l'image de saturation en oxygène est coloré en fonction du niveau de saturation en oxygène.

7. Système d'endoscope selon la revendication 5, **caractérisé en ce qu'**il inclut en outre :
un contrôleur d'affichage (72) permettant à la partie d'affichage d'afficher l'image spéciale avant détection de la lésion suspectée, et d'afficher l'image de saturation en oxygène lorsque la lésion suspectée est jugée comme étant dans l'état hydroxique.

8. Système d'endoscope selon la revendication 7, dans lequel le contrôleur d'affichage permet à la partie d'affichage d'afficher de manière temporaire l'image de saturation en oxygène entre le moment après détection de la lésion suspectée et le moment avant le jugement de la lésion suspectée comme étant dans l'état hydroxique.

9. Système d'endoscope selon la revendication 7, dans lequel avant l'affichage de l'image de saturation en oxygène sur la partie d'affichage, le contrôleur d'affichage affiche un message sur la partie d'affichage, et le message notifie que l'image de saturation en oxygène sera affichée sur la partie d'affichage.

10. Procédé destiné à fournir une assistance pour une endoscopie de diagnostic, **caractérisé en ce qu'**il comprend les étapes consistant à :
appliquer une lumière blanche à une région d'intérêt dans une cavité corporelle ;
produire une image de lumière normale sur la base d'un signal image émis à partir d'une région d'imagerie (60) durant l'éclairement avec la lumière blanche ;
appliquer une lumière mixte de la lumière blanche et de la lumière de bande étroite bleue à la région d'intérêt, la lumière blanche et la lumière de bande étroite bleue étant mélangées selon un rapport prédéterminé ;
produire une image présentant le bleu de mise en valeur sur la base d'un signal image émis à partir de la partie d'imagerie durant l'éclairement avec la lumière mixte ;
combiner l'image de lumière normale avec l'image présentant le bleu de mise en valeur afin de produire une image spéciale dans laquelle un vaisseau sanguin est mis en valeur ;
détecter une lésion suspectée dans l'image spéciale ;
appliquer deux types ou plus de lumière de bande étroite séquentiellement à la région d'intérêt, un coefficient d'absorption de chacune des lumières de bande étroite variant avec un niveau de saturation en oxygène ;
acquérir deux signaux de saturation en oxygène ou plus, à partir de signaux image, correspondant à la lumière de bande étroite respective, émis à partir de la partie d'imagerie durant l'éclairement séquentiel avec la lumière de bande étroite ;
obtenir un niveau de saturation en oxygène du sang dans le vaisseau sanguin à partir de l'amplitude d'un rapport entre les signaux de saturation en oxygène ;
produire une image de saturation en oxygène, qui est une image du vaisseau sanguin représenté par le niveau de saturation en oxygène ;
juger si le niveau de saturation en oxygène de la lésion suspectée est dans un état hypoxique dans les limites d'une plage prédéterminée, et
afficher l'image spéciale sur une partie d'affichage (14) avant détection de la lésion suspectée, et afficher l'image de saturation en oxygène sur la partie d'affichage lorsque la lésion suspectée est jugée comme étant dans l'état hydroxique.

11. Procédé selon la revendication 10, dans lequel l'image de saturation en oxygène est affichée de manière temporaire sur la partie d'affichage entre le moment après détection de la lésion suspectée et le moment avant le jugement de la lésion suspectée comme étant dans l'état hydroxique.
